# EUROPEAN PATENT APPLICATION

(11) **EP 2 305 092 A1**
(43) Date of publication of application: **06.04.2011**
(21) Application number: 10174135.3
(22) Date of filing: 26.08.2010
(51) Int. Cl.: A61B 1/00

(54) **Endoscope having optical fibers**

(30) Priority: 27.08.2009 JP 2009196514
(71) Applicant: Fujifilm Corporation, Minato-ku Tokyo (JP)
(72) Inventor: Ozaki, Takao, Kanagawa (JP); Kuroda, Osamu, Kanagawa (JP); Nakamura, Takayuki, Kanagawa (JP); Iida, Takayuki, Kanagawa (JP)
(74) Representative: Klunker . Schmitt-Nilson . Hirsch

(57) **Abstract**

An endoscope for image pickup of an object by receiving image light with an image sensor (56) includes an elongated tube (8). A fiber optic image guide (31) includes a plurality of optical fibers being bundled, and has a distal tip (48), is inserted through the elongated tube, for transmitting the image light focused on the distal tip by an objective lens system (30) in a proximal direction. A piezoelectric actuator (35) is disposed on an outer side of 10the fiber optic image guide, for periodically displacing the distal tip, wherein the image sensor picks up the image light from the fiber optic image guide for plural times in synchronism with displacement, to form one synthesized image. A hood device (16, 90) is mounted on a head assembly (15) of 15the elongated tube, and shiftable between a closed position for covering a distal end face of the head assembly and an open position for revealing the distal end face.

## Description

### BACKGROUND OF THE INVENTION

### 1. Field of the Invention

The present invention relates to an endoscope having optical fibers. More particularly, the present invention relates to an endoscope having optical fibers in which an elongated tube can have a reduced diameter, images of high image quality can be produced, and a head assembly of the elongate tube can be protected effectively.

### 2. Description Related to the Prior Art

An endoscope is an important medical instrument used in the field of the clinical medicine. Examples of the endoscope include primitive models such as a fiberscope or stomach camera, an electronic endoscope containing a CCD, and a capsule endoscope which is orally swallowed by a patient to retrieve an image.

In the field of endoscopic examination, extreme thinning of a tube for a reduced diameter to produce an ultra thin tube is desired very seriously for an elongated tube of the endoscope. Various ideas for the extreme thinning for a reduced diameter have been suggested for the purpose of imaging of various body parts in a narrow lumen, such as a pancreatic duct, bile duct, breast duct, terminal bronchioles, and the like.

The fiberscope is structurally suitable for the extreme thinning for a reduced diameter, because the image can be retrieved only by having a fiber optic image guide and an illuminating light guide. The fiber optic image guide transmits image light of the image of a body part or object of interest. The illuminating light guide applies light to the body part. However, cladding of an optical fiber bundle constituting the fiber optic image guide does not contribute to the transmission of the image light. There occurs a problem in that a pattern of mesh of a loss region due to the cladding appears locally within the image, and the image quality of the image will be low.

In view of this problem, U.S.P. No. 4,618,884 (corresponding to JP-A 60-053919) discloses the fiberscope. In a first embodiment of the document, a focusing lens system is disposed at a distal tip of the fiber optic image guide for focusing of image light on the distal tip. A piezoelectric actuator vibrates the focusing lens system to remove light component of a pattern of mesh from the image. The piezoelectric actuator vibrates the focusing lens system horizontally and vertically at a predetermined amount according to a pixel pitch of the pixels of the CCD or the optical fibers in the fiber optic image guide.

In a second embodiment of U.S.P. No. 4,618,884, the CCD is disposed at a distal end of the elongated tube without the fiber optic image guide. The focusing lens system in front of the CCD is vibrated in the same manner as its first embodiment. During the vibration, image light of the image is received on pixels of the CCD in a time division manner. Data are obtained, and written to a frame memory sequentially, to produce one frame of the image. Thus, high definition of the image can be obtained.

The focusing lens system has a larger diameter than the fiber optic image guide to ensure high brightness in the image. In U.S.P. No. 4,618,884, the focusing lens system is vibrated by the piezoelectric actuator. Even with the focusing lens system having the larger diameter than the fiber optic image guide, a further space is required for positioning a frame or retention mechanism for supporting the focusing lens system in a pivotally movable manner. The elongated tube must have a larger size in the radial direction. It follows that vibrating the focusing lens system with the piezoelectric actuator is inconsistent to the extreme thinning for a reduced diameter. A space required for positioning such a frame or retention mechanism is a serious problem in view of the extreme thinning for a reduced diameter of an order from tens of microns to a number of millimeters.

Although high definition of an image can be obtained from the second embodiment of U.S.P. No. 4,618,884, the extreme thinning for a reduced diameter is still impossible because the CCD is disposed in front of the elongated tube in addition to the focusing lens system.

Therefore, development of an endoscope system has been suggested in which a distal tip of a fiber optic image guide is displaced periodically by a piezoelectric actuator, and an object is imaged for plural times in synchronism with the displacement. One synthesized image is created from plural output images and also in consideration of information of shift amounts. This is for meeting purposes of achieving an ultra thin tube and obtaining images of high quality.

However, the suggestion of the development of the endoscope system lacks an air/water supply channel for washing the distal tip of the endoscope with air or water. This is for the reason of an ultra thin tube. For advance of the endoscope in a body, particles may stick on an imaging window of the elongated tube. Those will obstruct the imaging, and cannot be removed acceptably.

### SUMMARY OF THE INVENTION

In view of the foregoing problems, an object of the present invention is to provide an endoscope having optical fibers in which an elongated tube can have a reduced diameter, images of high image quality can be produced, and a head assembly of the elongated tube can be protected effectively.

In order to achieve the above and other objects and advantages of this invention, an endoscope for image pickup of an object by receiving image light with an image sensor is provided. An objective lens system is incorporated in an elongated tube, for entry of the image light from the object. A fiber optic image guide includes a plurality of optical fibers being bundled, and has a distal tip, is inserted through the elongated tube, for transmitting the image light focused on the distal tip by the objective lens system in a proximal direction. A piezoelectric actuator is disposed on an outer side of the distal tip, for periodically displacing the distal tip, wherein the image sensor picks up the image light from the fiber optic image guide for plural times in synchronism with displacement, to form one synthesized image. A hood device is mounted on a head assembly of the elongated tube, and shiftable between a closed position for covering a distal end face of the head assembly and an open position for revealing the distal end face. A moving mechanism moves the hood device from the closed position to the open position.

The hood device includes a transparent cover portion for covering the distal end face.

The cover portion is a resilient cap portion including an end opening. The hood device includes a sleeve, disposed about the distal tip to extend from the resilient cap portion in the proximal direction, moved between the open and closed positions by the moving mechanism, for narrowing the end opening in front of the distal end face when in the closed position, and for widening the end opening by pushing open the resilient cap portion with the distal end face when in the open position.

In a preferred embodiment, the cover portion is a cover door, and is moved by the moving mechanism for opening and closing.

Furthermore, a calibration chart is secured to a surface of the cover door directed in the proximal direction, for calibrating a shift amount of the fiber optic image guide.

The calibration chart includes plural stripe areas extending in a predetermined direction. The distal tip is displaced back and forth in a shift direction extending across the predetermined direction, and picks up an image of the calibration chart to form a test image, the shift amount being calibrated according thereto.

The distal tip is displaced by application of a driving voltage to the piezoelectric actuator. The test image is evaluated for calibration so as to determine an adjusted driving voltage for displacement of imaging.

The moving mechanism includes a control wire secured to the hood device. A wire moving device is disposed on a side of the proximal direction, for winding or unwinding the control wire.

The control wire is embedded partially in the elongated tube.

Consequently, an elongated tube can have a reduced diameter, images of high image quality can be produced, and a head assembly of the elongated tube can be protected effectively, because the hood device can operate for protecting the head assembly.

### BRIEF DESCRIPTION OF THE DRAWINGS

The above objects and advantages of the present invention will become more apparent from the following detailed description when read in connection with the accompanying drawings, in which:
Fig. 1 is a perspective view illustrating an endoscope system;
Fig. 2A is a perspective view illustrating a hood device in an endoscope;
Fig. 2B is a perspective view illustrating the same as Fig. 2A but in an open position;
Fig. 3 is a front elevation illustrating a head assembly of an endoscope of the endoscope system;
Fig. 4 is a vertical section illustrating the head assembly;
Fig. 5 is a perspective view illustrating a displacing device;
Fig. 6 is a front elevation illustrating a bundle of optical fibers of a fiber optic image guide;
Fig. 7 is a block diagram illustrating relevant elements in the endoscope system;
Fig. 8 is an explanatory view in a front elevation illustrating a relationship between an image transmitted by the core and a pixel of a CCD;
Fig. 9 is an explanatory view illustrating one example of displacement;
Fig. 10A is an explanatory view in a front elevation illustrating a two-dimensional path of a distal tip of one of the cores;
Fig. 10B is an explanatory view in a front elevation illustrating another two-dimensional path of the distal tip;
Fig. 11 is a block diagram illustrating relevant circuits for operation upon designating a composite imaging mode;
Fig. 12 is a timing chart illustrating a relationship between driving of the CCD, a piezoelectric control signal and an image synthesis signal;
Fig. 13 is a flow chart illustrating operation of the endoscope system;
Fig. 14 is a perspective view illustrating one preferred embodiment including a calibration chart inside a cover door;
Fig. 15 is a front elevation illustrating the cover door;
Fig. 16 is an explanatory view in elevations, illustrating the calibration chart;
Fig. 17 is an explanatory view illustrating a sequence of displacement of each of the cores in the calibration;
Fig. 18 is a graph illustrating a relationship between black density and a shift amount;
Fig. 19 is a table illustrating part images transmitted by cores in a reference position and set positions.

### DETAILED DESCRIPTION OF THE PREFERRED EMBODIMENT(S) OF THE PRESENT INVENTION

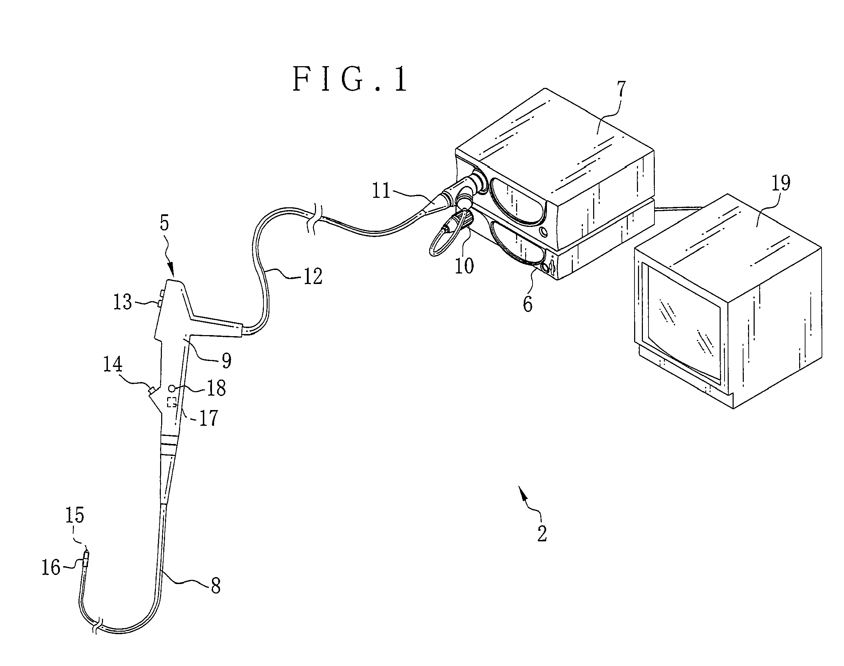

In Fig. 1, an endoscope system 2 includes an endoscope 5, a processing apparatus 6 and a light source apparatus 7. The endoscope 5 is used for imaging of various body parts in narrow lumens, for example, a pancreatic duct, bile duct, breast duct, terminal bronchioles, and the like. The endoscope 5 includes an elongated tube 8 or insertion tube, a handle 9, a first connector 10, a second connector 11 or coupler, and a universal cable 12. The elongated tube 8 is flexible and entered in a patient's body. The handle 9 is disposed at a proximal end of the elongated tube 8. The first connector 10 is plugged in the processing apparatus 6. The second connector 11 is plugged in the light source apparatus 7. The universal cable 12 extends from the handle 9 to the first connector 10 and the second connector 11.

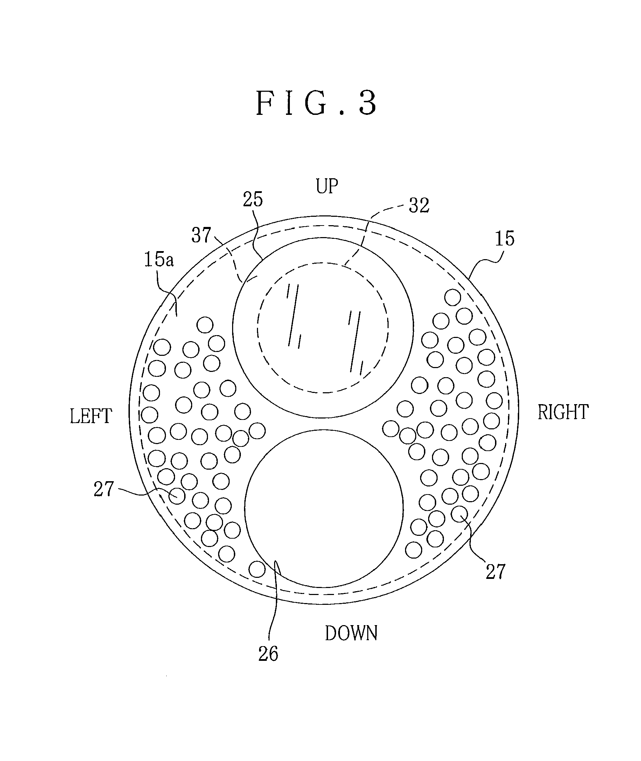

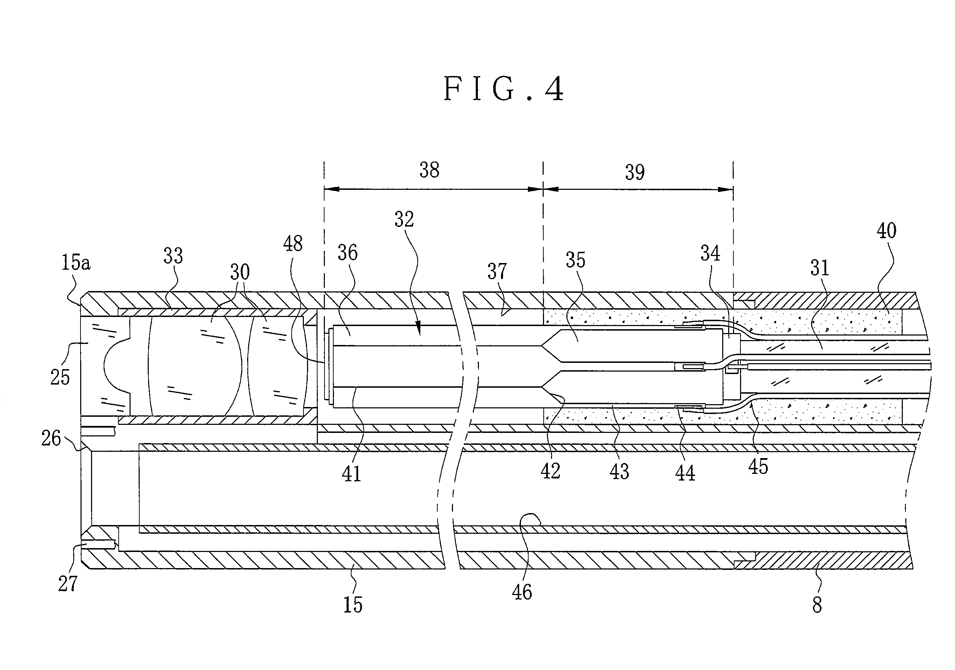

The elongated tube 8 has a thickness of 50 microns and outer diameter of 0.9 mm, and is formed from flexible material such as a Teflon (trade name), namely tetrafluoroethylene. A recording button 13 or release button among various buttons is disposed on the handle 9 for recording an endoscopic still image of a body part. An instrument opening 14 or forceps opening is formed on a side of the handle 9, and receives passage of an electrosurgical knife or other instruments for treatment. A head assembly 15 is disposed at a distal end of the elongated tube 8. An instrument opening 26 or forceps opening (See Fig. 3) is formed in the head assembly 15. A working channel 46 or forceps channel (See Fig. 4) is formed through the elongated tube 8, and extends from the instrument opening 14 to the instrument opening 26.

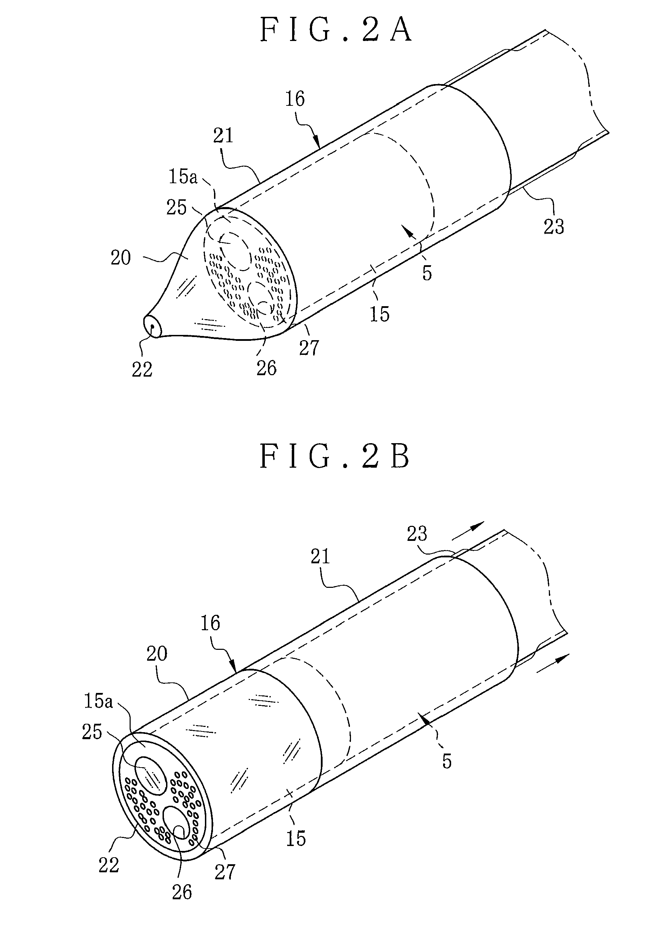

A hood device 16 or protection device or cover is secured to the head assembly 15. In Fig. 2, the hood device 16 includes a sleeve 21 and a resilient cap portion 20 with an easy open end. The resilient cap portion 20 has a decreasing width. An end opening 22 is formed in the resilient cap portion 20. Its size can be enlarged at the easy open end. Examples of materials of the resilient cap portion 20 are silicone rubber, polyurethane rubber and the like being transparent and colorless, and having biocompatibility and resiliency. A thickness of the sleeve 21 is very small. An inner bore of the sleeve 21 is substantially equal to an outer diameter of the head assembly 15. Its length is slightly larger than that of the head assembly 15. The sleeve 21 has a surface extending from that of the resilient cap portion 20 smoothly in a seamless manner, and can be handled together with the resilient cap portion 20. Examples of materials for the sleeve 21 are fluorocarbon resins and the like having biocompatibility and sufficient rigidity. Note that the material for the sleeve 21 may be the same as that for the resilient cap portion 20.

Two control wires 23 are secured to a proximal end of the sleeve 21 and positioned at an interval of 180 degrees. An example of the control wires 23 is plural coiled filaments of metal or the like having sufficient rigidity and sufficient flexibility. An intermediate portion of the control wires 23 is embedded in a sealing hole (not shown) formed in the elongated tube 8 of the endoscope 5. A length of an uncovered portion of the control wires 23 before the embedment is substantially equal to that of the resilient cap portion 20. A wall of the sealing hole is formed from flexible material, and has a slightly smaller diameter than an outer diameter of the control wires 23, to avoid entry of water or fluid into the elongated tube 8.

A winder 17 of Fig. 1 is incorporated in the elongated tube 8 and positioned near to the instrument opening 14. The control wires 23 are connected with the winder 17. The winder 17 is a reel mechanism or moving mechanism, and includes a spool and a spiral spring. Proximal ends of the control wires 23 are secured to the spool. The spiral spring biases the spool in a winding direction for the control wires 23. Also, a lock device is incorporated in the winder 17 for blocking rotation of the spool against the bias of the spiral spring. A winding button 18 is disposed near to the instrument opening 14, and depressed for disabling the lock device and releasing the spool from the regulation.

When the winding button 18 is operated manually, the lock device becomes unfastened. The bias of the spiral spring rotates the spool to wind the control wires 23. An amount of winding the control wires 23 about the spool is as much as a length of an uncovered portion of the control wires 23 before embedment in the sealing hole, namely the length of the resilient cap portion 20. In Fig. 2A, a distal end face 15a of the head assembly 15 is covered by the resilient cap portion 20 (closed position). The resilient cap portion 20 becomes peeled off in the proximal direction by enlarging the end opening 22 in Fig. 2B, to reveal the distal end face 15a (open position). When the resilient cap portion 20 is moved in the distal direction from the state of Fig. 2B, the spool rotates in an unwinding direction against the spiral spring until the lock device starts actuation. The control wires 23 are unwound from the winder 17 for return to the state of Fig. 2A. Note that an additional device can be installed for advancing and pressing the control wires 23 in the distal direction, to return from the state of Fig. 2B to the state of Fig. 2A in a semi-automatic manner.

For imaging, at first a doctor or operator enters the elongated tube 8 in a body by keeping the hood device 16 in the closed position. Upon reach to an object of interest, the control wires 23 are operated to peel off the resilient cap portion 20 to reveal the distal end face 15a of the head assembly 15 for the open position. After the imaging, the elongated tube 8 is moved back while the hood device 16 is in the open position. Thus, a mechanism for moving the hood device 16 from the open position of Fig. 2B to the closed position of Fig. 2A is unnecessary. Furthermore, it is possible to utilize contact of the hood device 16 with a wall in the patient's body with friction, and to pull the elongated tube 8 in the proximal direction to move the hood device 16 relatively in the distal direction for return to the closed position.

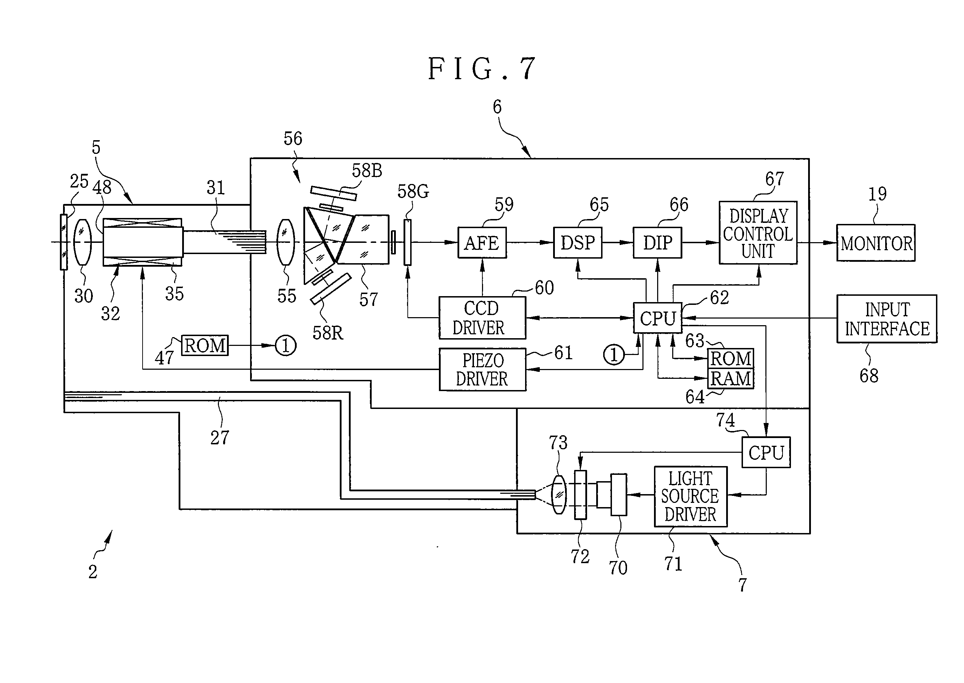

In Fig. 1, the processing apparatus 6 is connected with the light source apparatus 7 electrically, and controls operation of the constituents of the endoscope system 2. A connection cable 45 of Fig. 4 is inserted through the universal cable 12 and the elongated tube 8, and supplies the endoscope 5 with power from the processing apparatus 6. A displacing device 32 of Fig. 4 is also controlled by the processing apparatus 6. A fiber optic image guide 31 and a CCD group 58 are contained in the processing apparatus 6. The CCD group 58 includes CCDs 58B, 58G and 58R or image pickup devices. See Fig. 7. Image light of an image of a body part is transmitted by the fiber optic image guide 31, and received by the CCD group 58, which generates an image signal. The processing apparatus 6 processes the image signal in image processing, to create an image. A monitor display panel 19 is connected by use of a cable, and displays the image created by the processing apparatus 6.

The head assembly 15 has a wall constituted by a pipe of a stainless steel and has a thickness of 25 microns and an outer diameter of 0.8 mm. In Fig. 3, the distal end face 15a of the head assembly 15 is illustrated after removing the hood device 16. An imaging window 25 is disposed in an upper portion of the distal end face 15a. The instrument opening 26 is open in the distal end face 15a and disposed under the imaging window 25. A plurality of light guide devices 27 or illumination fiber optics are contained in the head assembly 15. Ends of the light guide devices 27 are positioned beside the imaging window 25 and the instrument opening 26 without bundling and packed randomly in a tube lumen inside the head assembly 15.

The instrument opening 26 has an outer diameter of 0.34 mm and an inner bore of 0.3 mm, and is an exit opening of the working channel 46. See Fig. 4. An example of material of a wall of the working channel 46 is polyimide. An example of the light guide devices 27 is an optical fiber having a diameter of 50 microns. The light guide devices 27 are inserted through the elongated tube 8 and the universal cable 12, and have a proximal end located in the second connector 11 or coupler. Light is entered through the proximal end of the light guide devices 27, is transmitted, and is applied to an object of interest through a distal end of the light guide devices 27 positioned within the distal end face 15a.

For the light guide devices 27, a plurality of optical fibers without bundling are inserted in the elongated tube 8. Adhesive agent in a fluid form is supplied into the head assembly 15 for adhesion of the light guide devices 27. It is possible according to requirements to polish the surface of the distal end of the light guide devices 27 after the adhesion, or to dispose a lighting window in front of the distal end of the light guide devices 27 to cover the same. Also, a coating of phosphor or other materials may be applied to the lighting window to diffuse the light.

In Fig. 4, the elements are depicted after removal of the hood device 16 in the same manner as Fig. 3. An objective lens system 30 is disposed behind the imaging window 25 together with the fiber optic image guide 31 and the displacing device 32. The displacing device 32 shifts the fiber optic image guide 31. A lens barrel 33 contains the objective lens system 30. A distal tip 48 for receiving light is positioned on a plane where image light of an image from an object is in focus. A diameter of the objective lens system 30 is 0.35 mm. An outer diameter of the lens barrel 33 is 0.4 mm. A length of the lens barrel 33 in the axial direction is 3.2 mm.

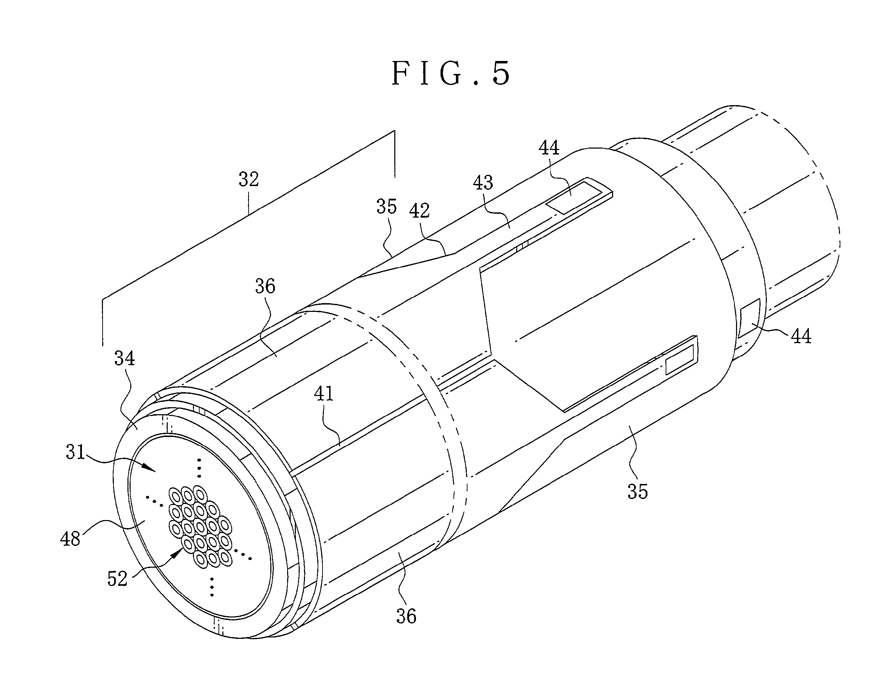

The fiber optic image guide 31 is a bundle of optical fibers with a diameter of 0.2 mm. See Fig. 5. The fiber optic image guide 31 extends through the elongated tube 8 and the universal cable 12, and has a proximal tip contained in the first connector 10. The fiber optic image guide 31 transmits image light of an object received from the objective lens system 30 through the distal tip 48 toward its proximal tip.

In Fig. 5, the displacing device 32 includes a support casing 34, a piezoelectric actuator material 35 and electrodes 36. The support casing 34 is a barrel or pipe of stainless steel, and has an outer diameter of 0.26 mm and an inner bore of 0.2 mm. The fiber optic image guide 31 is inserted in and fixed on the support casing 34. The piezoelectric actuator material 35 has a thickness of 15 microns, and is a coating applied to an outer surface of the support casing 34 in a cylindrical form. The electrodes 36 have a thickness of 5 microns, and are a coating about the piezoelectric actuator material 35.

The displacing device 32 is contained in a wall of the head assembly 15. A lumen 37 is defined between the outside of the displacing device 32 and the inside of the wall of the head assembly 15, and has a width of approximately 0.1 mm.

The displacing device 32 includes a shift mechanism 38 and a stationary section 39. The shift mechanism 38 is a portion of the displacing device 32 free from the wall of the head assembly 15 without fixing. The fiber optic image guide 31 is displaceable within the lumen 37 with respect to the stationary section 39. Adhesive agent 40 is used in the stationary section 39, and attaches the displacing device 32 to the inner wall of the head assembly 15. An area of the adhesive agent 40 extends from a proximal point of the displacing device 32 where the fiber optic image guide 31 appears to a point near to a distal end of the elongated tube 8. Lengths of the shift mechanism 38 and the stationary section 39 are respectively 4 mm and 1.9 mm in the axial direction. A length of filling of the adhesive agent 40 in the axial direction is 3.2 mm inclusive of the stationary section 39 and a distal portion of the elongated tube 8.

The electrodes 36 are arranged in the circumferential direction regularly at an angle of 90 degrees. The electrodes 36 are oriented with an inclination of 45 degrees relative to the vertical or horizontal direction of Fig. 3. Four grooves 41 are formed to extend in parallel with the axial direction, and define the electrodes 36 of two pairs. The electrodes 36 have a locally large width in the shift mechanism 38, as an interval between the electrodes 36 is only as great as the width of the grooves 41. In contrast, recesses 42 are defined with the electrodes 36 in the area of the stationary section 39, and extend in a symmetrical manner from the grooves 41. A narrow portion 43 of the electrodes 36 is defined by the recesses 42. The narrow portion 43 extends to the vicinity of the proximal end of the piezoelectric actuator material 35. The grooves 41 and the recesses 42 are formed by etching after applying a coating of the electrode material to the entire surface of the piezoelectric actuator material 35.

Pads 44 are disposed at proximal ends of the narrow portion 43. The connection cable 45 is connected with each of the pads 44. Also, the end of the support casing 34 has another one of the pads 44, with which the connection cable 45 is connected. Consequently, the support casing 34 operates as a common electrode for the piezoelectric actuator material 35.

The connection cable 45 has a cable diameter of 15 microns and an outer jacket diameter of 20 microns. The connection cable 45 is extended about the fiber optic image guide 31, inserted in the elongated tube 8 and the universal cable 12, and connected by the first connector 10 with the processing apparatus 6.

The two pairs of the electrodes 36 are supplied with voltages of opposite polarities with reference to a voltage applied to the support casing 34 as a common electrode. For example, let the support casing 34 have a potential of 0 V. An upper one of the electrodes 36 is supplied with +5 V. A lower one of the electrodes 36 is supplied with -5 V. The piezoelectric actuator material 35 under the electrodes 36 expands and contracts axially. In response to this, a portion of the shift mechanism 38 in front of the stationary section 39 displaces in the lumen 37 together with the distal tip 48 of the fiber optic image guide 31. It is possible to displace the shift mechanism 38 at a predetermined angle and amount by changing a combination of the electrodes 36 for powering and levels of the voltages.

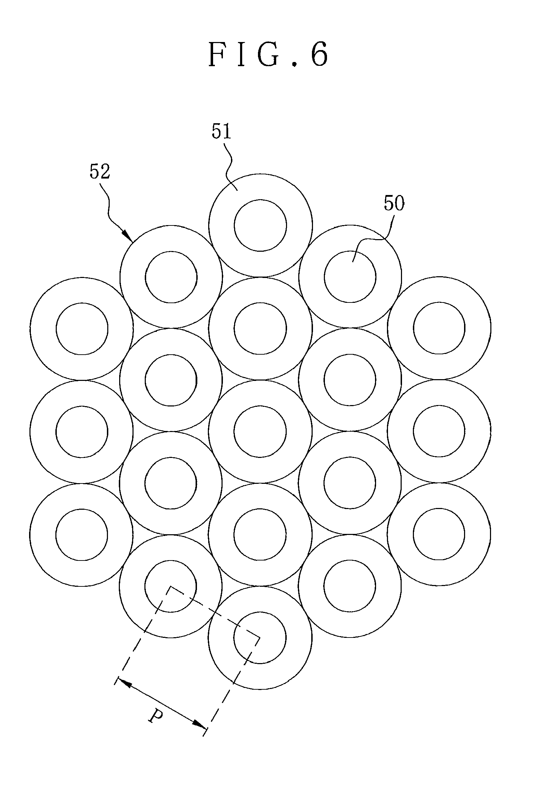

In Fig. 6, the fiber optic image guide 31 has plural optical fibers 52, for example 6,000 fibers, bundled with extreme tightness in an equilateral hexagonal form. Each of the optical fibers 52 includes a core 50 and a cladding 51. A diameter of the core 50 is 3 microns. A diameter of the cladding 51 is 6 microns. A pitch P of arrangement of the optical fibers 52 is 6 microns.

In Fig. 7, the processing apparatus 6 includes an enlarging lens system 55 and a three-CCD assembly 56. The enlarging lens system 55 is opposed to the proximal tip of the fiber optic image guide 31 extending to the outside of the first connector 10. The enlarging lens system 55 enlarges the object image from the fiber optic image guide 31 with a suitable magnification, and directs its image light to the three-CCD assembly 56.

The three-CCD assembly 56 is an image sensor disposed behind the enlarging lens system 55. A color separation prism 57 is combined with the CCD group 58 to constitute the three-CCD assembly 56 as well-known in the art. The color separation prism 57 includes three prism blocks and two dichroic mirrors disposed on optical faces of the prism blocks. The color separation prism 57 separates image light of a body part from the enlarging lens system 55 into light components of red, blue and green colors, which are directed to the CCD group 58. The CCD group 58 outputs an image signal according to light amounts of the light components from the color separation prism 57. Note that a CMOS image sensor may be used instead of the CCD.

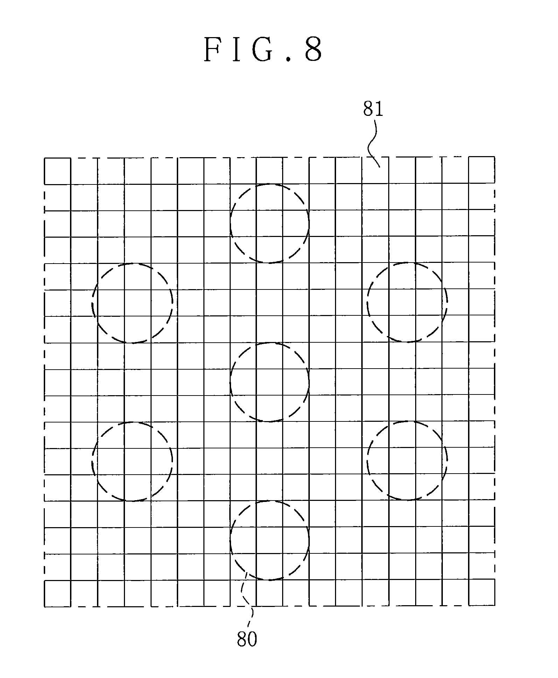

Image light of a part image 80 is transmitted by the core 50 of the fiber optic image guide 31. There are pixels 81 arranged on the image pickup surface of the CCD group 58. In Fig. 8, the part image 80 is viewed in a state projected to the image pickup surface of the CCD group 58. A center of the part image 80 substantially coincides with the center of a set of nine of the pixels 81. The proximal tip of the fiber optic image guide 31 is positioned relative to the color separation prism 57 and the CCD group 58 so as to correlate the part image 80 with the pixels 81 in the depicted state.

In Fig. 7, an analog front end 59 or AFE is supplied with the image signal from the CCD group 58. The analog front end 59 includes a correlated double sampling circuit or CDS circuit, an automatic gain control circuit or AGC circuit, and an A/D converter. The CDS circuit processes the image signal from the CCD group 58 in the correlated double sampling, and eliminates noise generated by the CCD group 58, such as reset noise, amplification noise and the like. The AGC circuit amplifies the image signal with a predetermined signal gain after noise elimination in the CDS circuit. The A/D converter converts the amplified image signal from the AGC circuit into a digital signal with a predetermined number of bits. A digital signal processor 65 or DSP has a frame memory (not shown) to which the digital form of the image signal from the A/D converter is written.

A CCD driver 60 generates drive pulses for the CCD group 58 and a sync pulse for the analog front end 59. The drive pulses include a vertical/horizontal scan pulse, electronic shutter pulse, reading pulse, reset pulse and the like. The CCD group 58 responds to the drive pulses from the CCD driver 60, takes an image, and outputs an image signal. Components included in the analog front end 59 are operated according to the sync pulse from the CCD driver 60. Note that the CCD driver 60 and the analog front end 59 are connected with the CCD 58G in the drawing, but also connected with the CCDs 58R and 58B.

A piezoelectric driver 61 is connected by the connection cable 45 with the electrodes 36 and the support casing 34. A CPU 62 controls the piezoelectric driver 61 to supply the piezoelectric actuator material 35 with voltage.

The CPU 62 controls the entirety of the processing apparatus 6. The CPU 62 is connected with various elements by a data bus (not shown), address bus, control lines and the like. A ROM 63 stores data (such as graphic data) and programs (operation system and application programs) for controlling the processing apparatus 6. The CPU 62 reads the program and data required for the purpose from the ROM 63. A RAM 64 is a working memory with which the CPU 62 performs tasks with data for operation by running the program. An input interface 68 is also associated with the CPU 62. The CPU 62 is supplied with information related to the examination by the input interface 68 or the LAN (local area network) or other networks, the information including a date and time of the examination, personal information of a patient, doctor's name, other text information, and the like. The CPU 62 writes the information to the RAM 64.

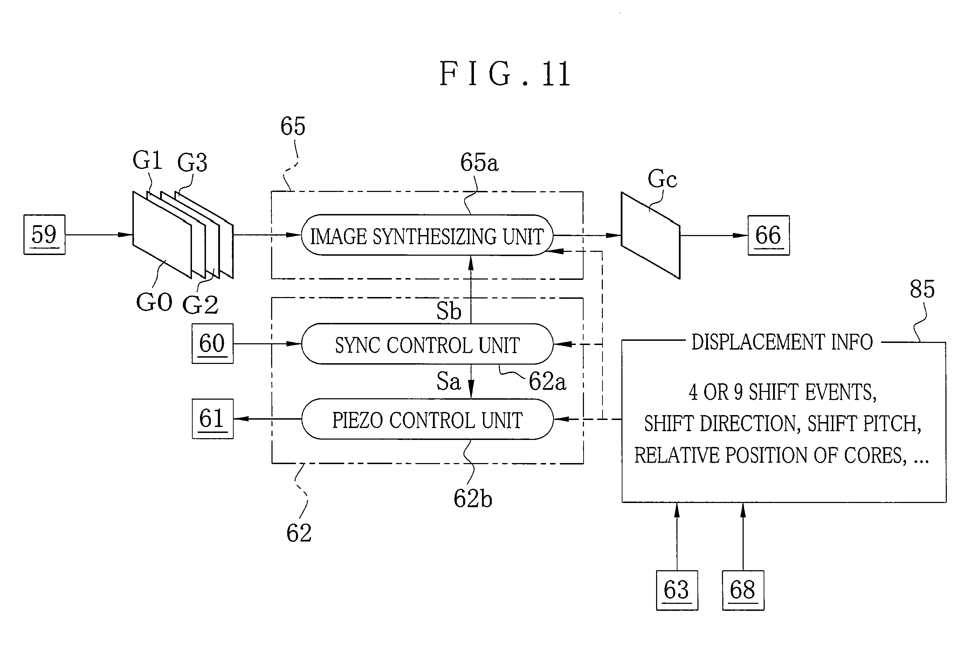

The digital signal processor 65 reads an image signal produced by the analog front end 59 from the frame memory. The digital signal processor 65 processes the image signal in processing of various functions, such as color separation, color interpolation, gain correction, white balance adjustment, gamma correction and the like, and produces an image of one frame. Also, the digital signal processor 65 has an image synthesizing unit 65a. See Fig. 11. When a composite imaging mode (to be described later) is selected, the image synthesizing unit 65a outputs one synthesized image of a high definition by combining plural images obtained in one two-dimensional shift sequence. To this end, plural frame memories are incorporated in the digital signal processor 65. A digital image processor 66 includes a frame memory (not shown), to which the image or synthesized image from the digital signal processor 65 is written.

The digital image processor 66 is controlled by the CPU 62 for image processing. The digital image processor 66 reads images from the frame memory after processing in the digital signal processor 65. Examples of functions of the image processing in the digital image processor 66 are electronic zooming, color enhancement, edge enhancement and the like. A display control unit 67 is supplied with data of the image processed by the digital image processor 66.

The display control unit 67 has a VRAM for storing the processed image from the digital image processor 66. The display control unit 67 receives graphic data read by the CPU 62 from the ROM 63 and the RAM 64. Examples of the graphic data include data of a mask for display of an active pixel area by masking an inactive area in the endoscopic image, text information such as an examination date, patient's name, and doctor's name, and data of graphical user interface (GUI), and the like. The display control unit 67 processes the image from the digital image processor 66 in various functions of display control, the functions including superimposition of the mask, the text information and the GUI, graphic processing of data for display on the display panel 19, and the like.

The display control unit 67 reads an image from the VRAM, and converts the image into a video signal suitable for display on the display panel 19, such as a component signal, composite signal or the like. Thus, the endoscopic image is displayed by the display panel 19.

The input interface 68 is a well-known input device, of which examples are an input panel on a housing of the processing apparatus 6, buttons on the handle 9 of the endoscope 5, mouse, keyboard, or the like. The CPU 62 operates relevant elements in the processing apparatus 6 in response to an input signal from the input interface 68.

The processing apparatus 6 also includes an image compression device, a media interface and a network interface. The image compression device compresses images in a format of compression, for example JPEG format. The media interface operates in response to an input from the recording button 13, and records the compressed images to a recording medium such as a CF card, MO (optical magnetic disk), CD-R and other removable media. The network interface transmits or receives various data by use of the LAN or other networks. Those are connected to the CPU 62 by a data bus or the like.

A light source 70 is incorporated in the light source apparatus 7. Examples of the light source 70 are a xenon lamp, white LED and the like which generate light of a broad band of the wavelength from red to blue, for example, with a wavelength of 480-750 nm. A light source driver 71 drives the light source 70. An aperture stop device 72 is disposed in front of the light source 70, and adjusts an amount of incident light. A condenser lens 73 condenses the light passed through the aperture stop device 72, and directs the light to the distal end of the light guide devices 27. A CPU 74 communicates with the CPU 62 of the processing apparatus 6, and controls the light source driver 71 and the aperture stop device 72.

There are two imaging modes including a normal imaging mode without operating the displacing device 32 and a composite imaging mode in operation of the displacing device 32. In the composite imaging mode, the number of shift events is changeable between four and nine. The input interface 68 can be operated to change over the imaging modes and set the number of shift events.

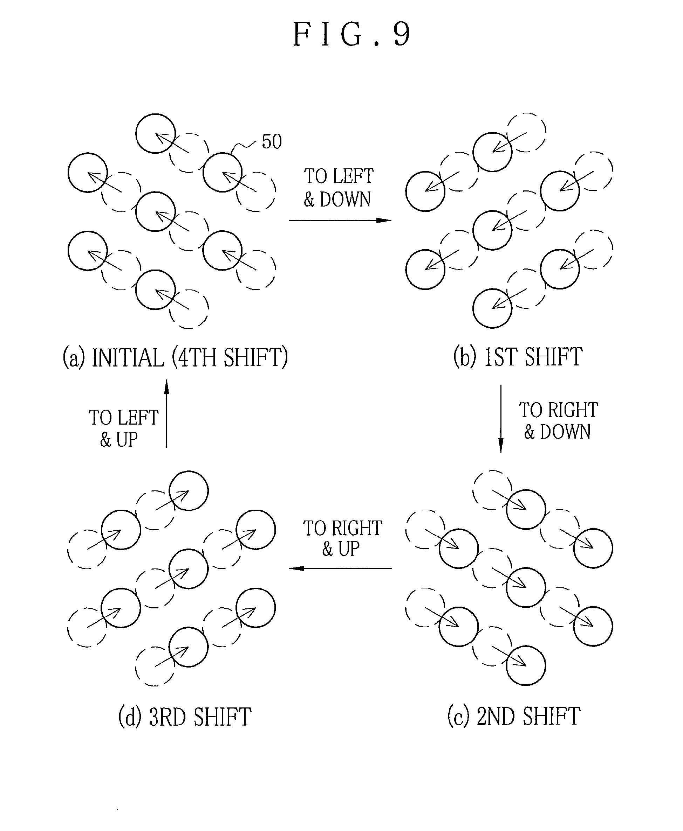

When the composite imaging mode is selected to set the four shift events, the piezoelectric driver 61 drives the shift mechanism 38 of the displacing device 32 to displace the distal tip 48 of the fiber optic image guide 31 as illustrated in Fig. 9. At first, the shift mechanism 38 displaces the distal tip 48 laterally from the initial position of (a) leftwards and downwards with an inclination of 30 degrees, and with an amount half as much as the pitch P of arrangement of the optical fibers 52. The shift mechanism 38 sets the distal tip 48 in a set position of (b) with a first shift event. Then the distal tip 48 is displaced at the same amount in the rightward and downward direction, and set in a set position of (c) with a second shift event. In sequence, the distal tip 48 is displaced at the same amount in the rightward and upward direction, and set in a set position of (d) with a third shift event. Then the distal tip 48 is displaced at the same amount in the leftward and upward direction, and set in an initial position of (a) with a fourth shift event by way of the initial position. The shift mechanism 38 is stopped in the set positions stepwise by the piezoelectric driver 61. Note that the solid line in the drawing indicates an actual position of the core 50 at the distal tip 48. The broken line indicates a previous position of the core 50 before the actual position.

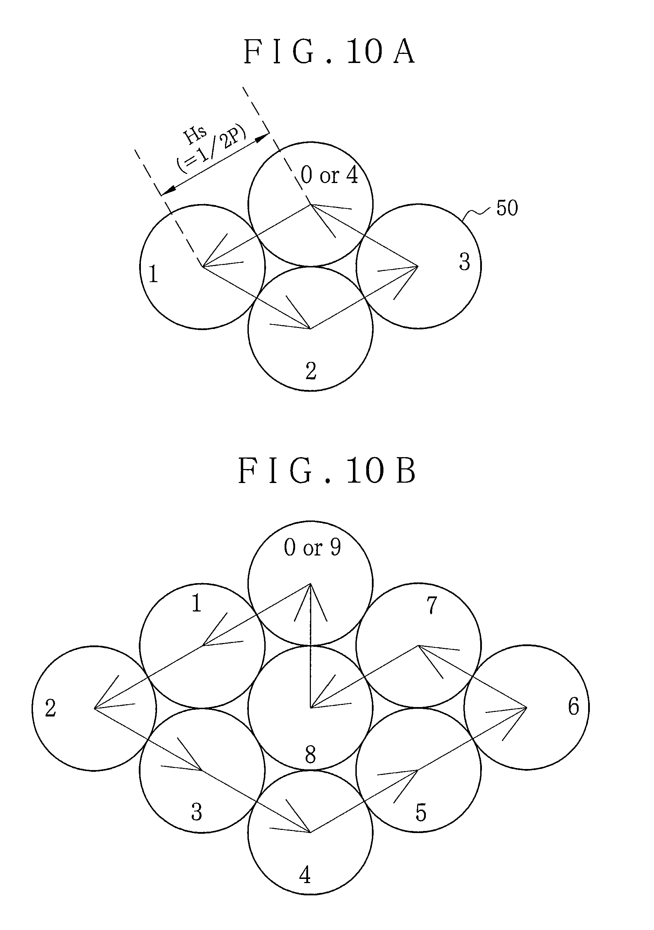

The core 50 in the distal tip 48 of the fiber optic image guide 31 repeatedly displaces in a composite sequence from the initial position of (a) to the set positions of (b), (c) and (d) then to the initial position of (a). The distal tip 48 shifts in a two-dimensional path of a polygonal shape of a rhombus of Fig. 10A to compensate for a loss region due to the cladding 51 in transmitting image light according to the initial position of (a).

Let a number of shift events be nine (9). In Fig. 10B, a two-dimensional path according to the nine shift events is illustrated. The number of shift events in each of the directions is one larger than that according to the mode of the four shift events. Note that a lateral direction from the seventh set position to the eighth set position is downward in contrast with the upward direction from the sixth set position to the seventh set position. A lateral direction from the eighth set position to the initial position or the ninth set position is upward with an angle of 90 degrees. In a manner similar to the mode of the four shift events, the two-dimensional path according to the nine shift events is in a shape to compensate for a loss region of the cladding 51 in transmitting image light according to the initial position. Furthermore, the distal tip 48 is displaced to the positions of the second, fourth and sixth set positions, which are the same as initial positions of three adjacent cores among the cores 50.

In Fig. 11, the composite imaging mode is designated. A sync control unit 62a and a piezoelectric control unit 62b are started in the CPU 62 of the processing apparatus 6. According to displacement information 85, the image synthesizing unit 65a of the digital signal processor 65 cooperates with the sync control unit 62a and the piezoelectric control unit 62b to perform various tasks.

The displacement information 85 is data related to shift events of the shift mechanism 38 of the displacing device 32. Examples of the data are a number of shift events, shift direction, shift pitch, relative positions of the pixels 81 of the CCD group 58 and the part image 80 transmitted through the core 50 of the fiber optic image guide 31 of Fig. 8. The data of the number of shift events is generated by the input interface 68. The ROM 63 stores basic datasets of the shift direction, shift pitch, relative positions of the pixels 81 and the part image 80. Any of those is read from the ROM 63 to the image synthesizing unit 65a, the sync control unit 62a and the piezoelectric control unit 62b.

The sync control unit 62a receives information of the drive pulses for the CCD group 58 from the CCD driver 60, and sends the piezoelectric control signal Sa to the piezoelectric control unit 62b and the image synthesis signal Sb to the image synthesizing unit 65a. The piezoelectric control unit 62b controls the piezoelectric driver 61 for fine displacement in synchronism with the piezoelectric control signal Sa. Similarly, the image synthesizing unit 65a performs a task of image synthesis in synchronism with the image synthesis signal Sb. Pixels of images G0, G1, G2 and G3 obtained from the set positions (in the mode of the four shift events) are mapped in accordance with the set positions, to create one synthesized image Gc.

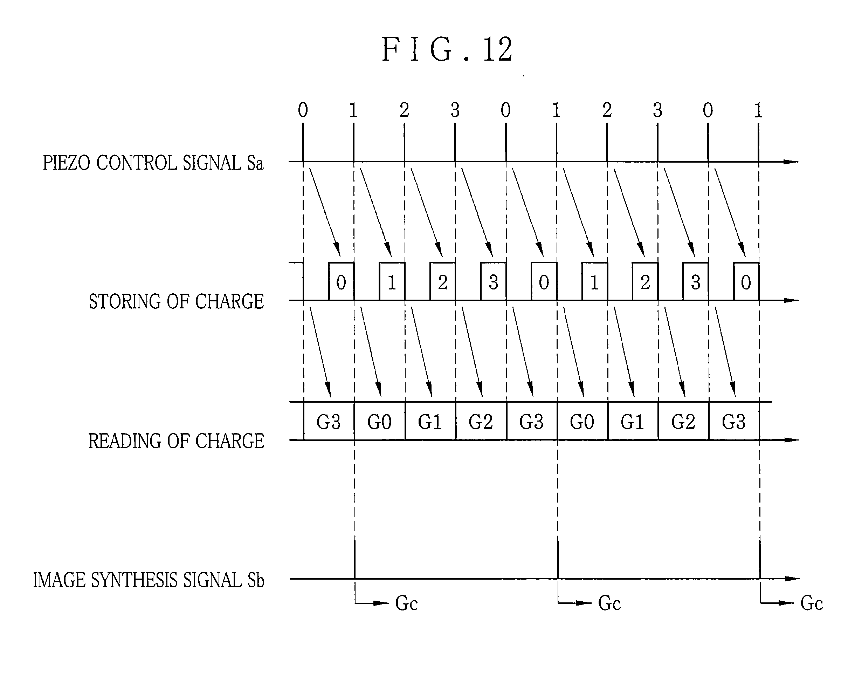

In Fig. 12, a mode of the four shift events is illustrated. Immediately after completing storing of the charge in the CCD group 58, the sync control unit 62a generates a piezoelectric control signal Sa. This is when the signal charge of one frame is read to a vertical transfer path from the pixels 81 of the CCD group 58 (or when a reading pulse is output by the CCD driver 60 to the CCD group 58). Also, the sync control unit 62a generates an image synthesis signal Sb upon completion of reading the charge of the CCD group 58 in correspondence with the image G3 obtained at the third set position. The operation of reading the charge is a sequence of CCD operation inclusive of reading the signal charge from the pixels 81 of the CCD group 58 to the vertical path, and vertical transfer, horizontal transfer and an output of an image signal of one frame.

The piezoelectric driver 61 in response to the piezoelectric control signal Sa supplies the piezoelectric actuator material 35 with a predetermined voltage, to displace the shift mechanism 38 from a previous set position to a present set position. Shift time from an output of the piezoelectric control signal Sa from the sync control unit 62a to the piezoelectric driver 61 until shift of the shift mechanism 38 to a succeeding set position is shorter than clearing time from completion of previous storing of charge in the CCD group 58 until a start of succeeding storing of charge. Thus, succeeding storing of charge is always started while the shift mechanism 38 is kept set in the succeeding set position by the piezoelectric driver 61.

The image synthesizing unit 65a in response to the image synthesis signal Sb reads images G0-G3 obtained from the set positions of displacement. The image synthesizing unit 65a maps pixels of the images G0-G3 according to the set positions of displacement, and outputs a synthesized image Gc. It is further possible to interpolate the pixels to process the images G0-G3 or synthesized image Gc in the course of the synthesis.

In the synthesized image Gc, a loss region due to the cladding 51 in transmitting image light can be compensated for in a visible manner. Pixel values of the pixels of the portions are directly derived from the object image without approximation or interpolation of adjacent pixels within one frame. Consequently, the number of pixels is higher than that in images obtained from the normal imaging mode or according to each one of the set positions of displacement, to produce the image in a very fine quality. Note that the image quality is higher in images obtained according to the nine shift events than images of the four shift events.

It is to be noted that the images G0-G3 are different part images 80 with differences in the set position by displacement. The part image 80 at the distal tip 48 is only shifted by keeping a proximal tip of the fiber optic image guide 31 stationary. No change occurs in the relative position between the proximal tip of the fiber optic image guide 31 and an image pickup surface of the CCD group 58. There are no apparently distinct feature between data output according to the pixels 81 even with the various set positions. For example, the part image 80 of a position in the image G0 is different from the part image 80 of the same position in the image G1 in relation to the set position. However, those are recorded commonly by the pixels 81 on the CCD group 58. Accordingly, the image synthesizing unit 65a determines original pixels of pixel values of the images among the pixels 81 by mapping on the basis of the relative position of the part image 80 of the displacement information 85 and the pixels 81, to carry out the pixel interpolation.

The operation of the endoscope system 2 of the above construction is described now. To observe a body part of a patient endoscopically, a doctor or operator connects the endoscope 5 to the processing apparatus 6 and the light source apparatus 7, which are turned on and powered. The input interface 68 is manually operated to input information related to the patient, to start examination.

After instructing the start, the doctor or operator enters the elongated tube 8 in the body. Light from the light source apparatus 7 is applied to body parts, while he or she observes an image on the display panel 19 from the CCD group 58 of the image sensor.

To introduce the elongated tube 8 in the body, the resilient cap portion 20 of the hood device 16 covers the distal end face 15a as illustrated in Fig. 2A. Particles or foreign material present in the body will not stick on the distal end face 15a because of the closed position of the resilient cap portion 20 for protection. As the resilient cap portion 20 is formed from colorless transparent material, an object can be imaged safely through the same. When fine imaging of the object of interest starts upon reach of the head assembly 15 thereto, the doctor or operator depresses the winding button 18 to actuate the winder 17. The hood device 16 is moved in a proximal direction as illustrated in Fig. 2B to peel off the resilient cap portion 20 for the open position. The distal end face 15a is revealed to ensure a field of view with more clarity than in the closed position of Fig. 2A of covering of the resilient cap portion 20.

An image signal is generated by the CCD group 58, processed by the analog front end 59 for various functions of processing, and input to the digital signal processor 65. The digital signal processor 65 processes the image signal for various functions of signal processing, to produce an image. The image from the digital signal processor 65 is output to the digital image processor 66.

The digital image processor 66 is controlled by the CPU 62 and processes the image from the digital signal processor 65 for various functions of image processing. The image is input by the digital image processor 66 to the display control unit 67. According to the graphic data from the CPU 62, the display control unit 67 carries out control for display. Thus, the image is displayed on the display panel 19 as an endoscopic image.

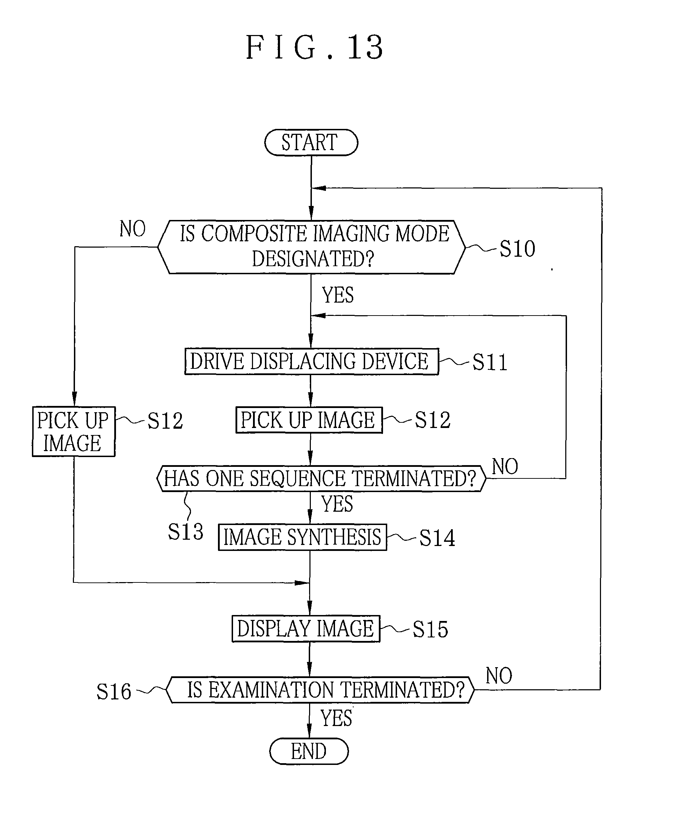

In Fig. 13, a composite imaging mode is designated (yes at the step S10). The sync control unit 62a and the piezoelectric control unit 62b are ready in the CPU 62 in the processing apparatus 6. According to the displacement information 85 and information of drive pulses from the CCD driver 60 for the CCD group 58, the sync control unit 62a sends the piezoelectric control signal Sa to the piezoelectric control unit 62b, and sends the image synthesis signal Sb to the image synthesizing unit 65a.

The operation of the piezoelectric driver 61 is controlled by the piezoelectric control unit 62b upon receiving the piezoelectric control signal Sa. The piezoelectric driver 61 applies a predetermined voltage to the piezoelectric actuator material 35. Thus, the shift mechanism 38 displaces at a predetermined angle and pitch according to the designated number of the shift events. See the step S11. At each time that the shift mechanism 38 is retained in one of the set positions, charge is stored in the CCD group 58. The part image 80 of a body part is picked up by the pixels 81 through the fiber optic image guide 31 in the step S12. A sequence including the steps S11 and S12 is repeated (no at the step S13) until the end of one two-dimensional shift sequence by shift of the shift mechanism 38 from the initial position and again to the same position.

When one two-dimensional shift sequence is terminated (yes at the step S13), image synthesis is carried out by the image synthesizing unit 65a upon receiving the image synthesis signal Sb, to produce a synthesized image at the step S14 from the images obtained according to the set positions of fine displacement. After data of the synthesized image is processed in the digital image processor 66 and the display control unit 67, the synthesized image is displayed on the display panel 19 at the step S15. In contrast, when the normal imaging mode is designated, an image is picked up in the step S12 but without carrying out the sequence of the steps S11 and S14. Those steps are repeated until a command signal for terminating the examination is input (yes at the step S16).

As described heretofore, the distal end face 15a is covered and protected in the course of advance of the elongated tube 8. The distal end face 15a is revealed by displacing the hood device 16 for imaging of an object of interest endoscopically. Thus, the head assembly 15 can be kept clean without depositing of foreign material or particles in the course of advance of the elongated tube 8. A field of view in the imaging can be maintained.

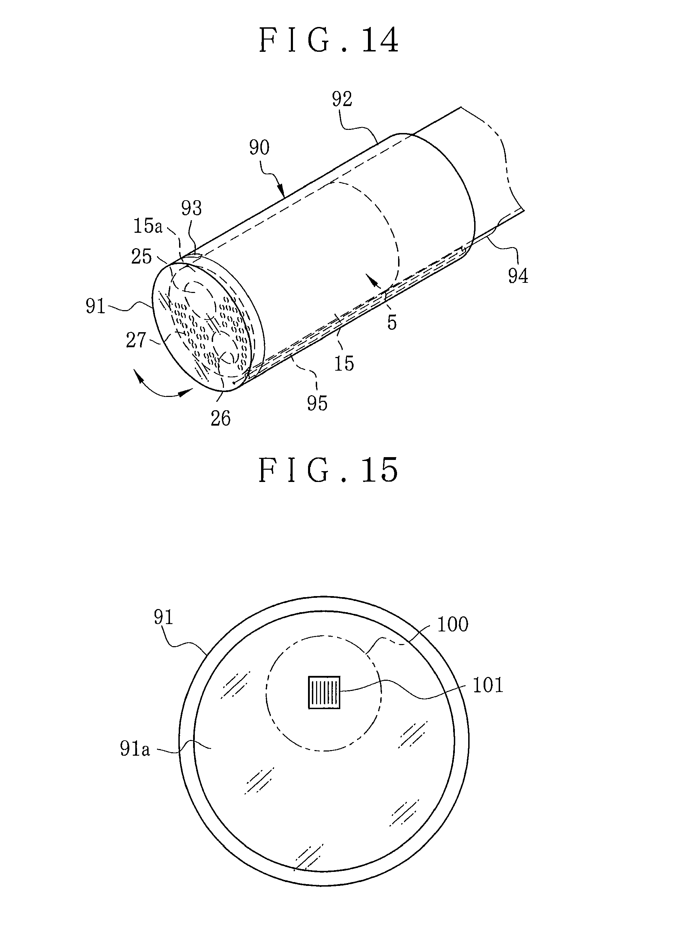

It is possible to extend the sleeve 21 to the vicinity of the root of the handle 9 and in a form of a bag or tube. No embedment of the control wires 23 in the elongated tube 8 is required. The control wires 23 are inserted through a groove or channel formed in the sleeve 21. See a groove 95 or channel in Fig. 14. This makes it unnecessary to form a sealing hole in the elongated tube 8 for the control wires 23. The elongated tube 8 can be protected by the sleeve 21.

Another preferred hood device 90 or protection device or cover is illustrated in Fig. 14. A cover door 91 is included in the hood device 90 instead of the resilient cap portion 20. Examples of materials of the cover door 91 are various transparent colorless materials in the manner of the resilient cap portion 20. The hood device 90 includes a sleeve 92 and a hinge 93 for securing the cover door 91 to the sleeve 92. The cover door 91 is movable pivotally about the hinge 93 between open and closed positions in the arrow direction. A torsion coil spring (not shown) is incorporated in the hinge 93 and biases the cover door 91 in an opening direction. In the drawing, the cover door 91 is in the closed position against the bias of the torsion coil spring.

A control wire 94 as moving mechanism is secured to a rear surface 91a of the cover door 91 opposite to the hinge 93. See Fig. 15. The control wire 94 has coiled filaments of metal similar to the control wires 23. The groove 95 or channel is formed in the sleeve 92. The control wire 94 extends through the groove 95, appears in a proximal end of the sleeve 92 to the outside, and is embedded in a sealing hole of the elongated tube 8. As the control wire 94 passes through the elongated tube 8, its proximal end is secured to a spool of a winder (not shown) disposed near to the instrument opening 14.

The winder includes an operation button in addition to the above-described elements in the winder 17, the operation button for freely rotating the spool by release from the bias of the spiral spring. The force of the bias of the spiral spring is higher than that of the torsion coil spring of the hinge 93. When the lock device is disabled by a setting of the winding button 18, rotation of the spool pulls the control wire 94 in a proximal direction, to close the cover door 91 against the bias of the torsion coil spring.

When the operation button for free rotation of the spool is depressed, application of pulling force to the control wire 94 is discontinued, to open the cover door 91 with the bias of the tension coil spring. When the lock device is actuated while the cover door 91 is open or closed, the cover door 91 becomes stopped during the actuation. The maximum opening angle of the cover door 91 is 90 degrees or larger than 90 degrees. A length of the control wire 94 and an amount of winding of the control wire 94 about the spool are adjusted for setting the maximum opening angle.

To enter the elongated tube 8 in a patient's body, a doctor or operator depresses the winding button 18 to close the cover door 91. The distal end face 15a is covered by the cover door 91 as depicted in the drawing. To start imaging of an object of interest, he or she depresses the operation button for freely rotating the spool. The cover door 91 is opened to reveal the distal end face 15a. Accordingly, the same effect as the first embodiment can be obtained.

Note that the cover door 91 may be directly secured to the head assembly 15 except for the sleeve 92. Also, it is possible to dispose the hinge 93 close to the instrument opening 26, and dispose a portion of the control wire 94 for attachment to the rear surface 91a of the cover door 91 close to the imaging window 25. Also, a portion of the control wire 94 for attachment to the rear surface 91a of the cover door 91 can be defined near to the hinge 93 or the like for the purpose of keeping the control wire 94 out of the field of view upon opening the cover door 91.

In Fig. 15, a calibration chart 101 for a shift amount is secured to a part, for example central part, of a rear area 100 (indicted by the phantom line) of the rear surface 91a of the cover door 91 opposed to an imaging window. Black and white areas 110a and 110b as stripe areas are arranged on the calibration chart 101 as will be described later. The hood device 90 is fixedly secured to the head assembly 15 by aligning a shift direction of the fiber optic image guide 31 with the width direction of the black and white areas 110a and 110b. There is a positioning structure for positioning the hood device 90. Examples of the positioning structure can include a combination of a groove in an inner surface of the sleeve 92 in the hood device 90 and a projection on the head assembly 15 for engagement with the groove in the sleeve 92, and a combination of a projection on an inner surface of the sleeve 92 in the hood device 90 and a groove in the head assembly 15 for engagement with the projection in the sleeve 92, and a combination of a male thread on the head assembly 15 and a female thread in the sleeve 92.

Due to specificity in the fiber optic image guide 31 and the piezoelectric actuator material 35 for shifting the fiber optic image guide 31, failure may occur in shifting of the distal tip 48 of the fiber optic image guide 31 at a predetermined shift amount even if the piezoelectric actuator material 35 is driven properly. An error in the image registration will occur in forming the synthesized image Gc, which will have an artifact. To prevent such a problem, calibration of the shift amount is carried out by use of the calibration chart 101.

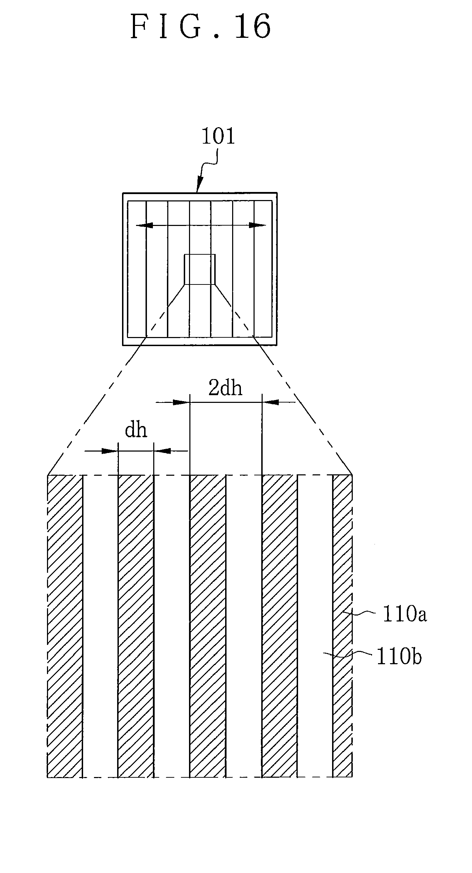

In Fig. 16, the calibration chart 101 has a multi-stripe pattern of the black and white areas 110a and 110b arranged alternately as stripe areas, in parallel and with an equal width dh. The black areas 110a are hatched in the drawing in contrast with the white areas 110b. A pitch width 2dh as a sum of widths of one of the black areas 110a and one of the white areas 110b is a constant value times the constant shift amount Hs, namely the shift amount in the direction of 30 degrees. In short, 2dh = kh.Hs where kh is the constant value. The constant value kh is determined according to the magnification of the objective lens system 30 and a distance between the distal end face 15a and the rear surface 91a of the cover door 91 in the closed position. The constant value kh is so defined that the pitch width 2dh of the black and white areas 110a and 110b on the plane of the CCD group 58 becomes equal to the shift amount Hs when the rear surface 91a of the cover door 91 in the closed position is opposed to the distal end face 15a.

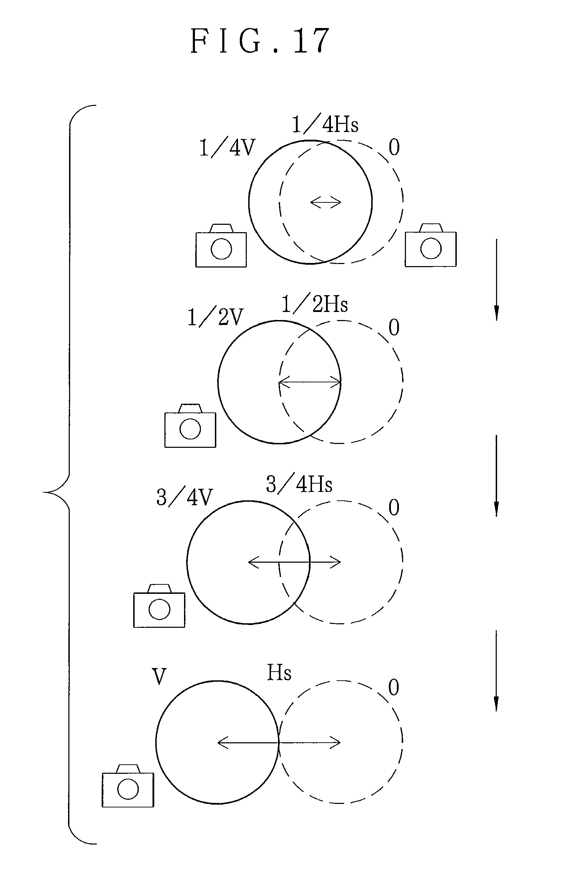

In Fig. 17, calibration of a shift amount is illustrated. The piezoelectric driver 61 is controlled by the CPU 62 and drives the piezoelectric actuator material 35 cyclically at a driving voltage V of a default value and at a driving voltage m/n.V, for example, 1/4.V, 1/2.V, 3/4.V and the like. The piezoelectric actuator material 35 is returned to the state before the shift (state of zero (0) indicated by the broken line) at each time. The default driving voltage V is as high a voltage level as to displace the distal tip 48 of the fiber optic image guide 31 with a predetermined shift amount by driving the piezoelectric actuator material 35. The distal tip 48 of the fiber optic image guide 31 is caused by the piezoelectric actuator material 35 with the driving voltage V to displace at the shift amount Hs in a direction of 30 degrees. When the driving voltage m/n.V is applied to the piezoelectric actuator material 35, the distal tip 48 displaces at a shift amount m/n.Hs. If there is failure of driving the piezoelectric actuator material 35, the shift amount of the distal tip 48 becomes different from the shift amount m/n.Hs.

The distal tip 48 of the fiber optic image guide 31 moves back and forth by a stepwise increase in the shift amount between a reference position of the broken line and one of the positions distant at each of shift amounts from the reference position. The time required for return from the previous set position to the reference position and for shift to a new set position is equal to the time required for shift from the previous set position to a new set position in the above described composite imaging mode. In short, the distal tip 48 of the fiber optic image guide 31 is displaced at a driving frequency equal to that in the composite imaging mode.

The CCD group 58 picks up an image of the calibration chart 101 initially in the reference position for one time and then in each of the set positions, as indicated by a sign of a camera. The CPU 62 detects black density of a local area or an entire area of a test image obtained by the CCD group 58 from the calibration chart 101. According to the black density, a shift amount or the driving voltage for the piezoelectric actuator material 35 is calibrated. Light generated by the light source 70 in the light source apparatus 7 is used in a manner similar to the imaging.

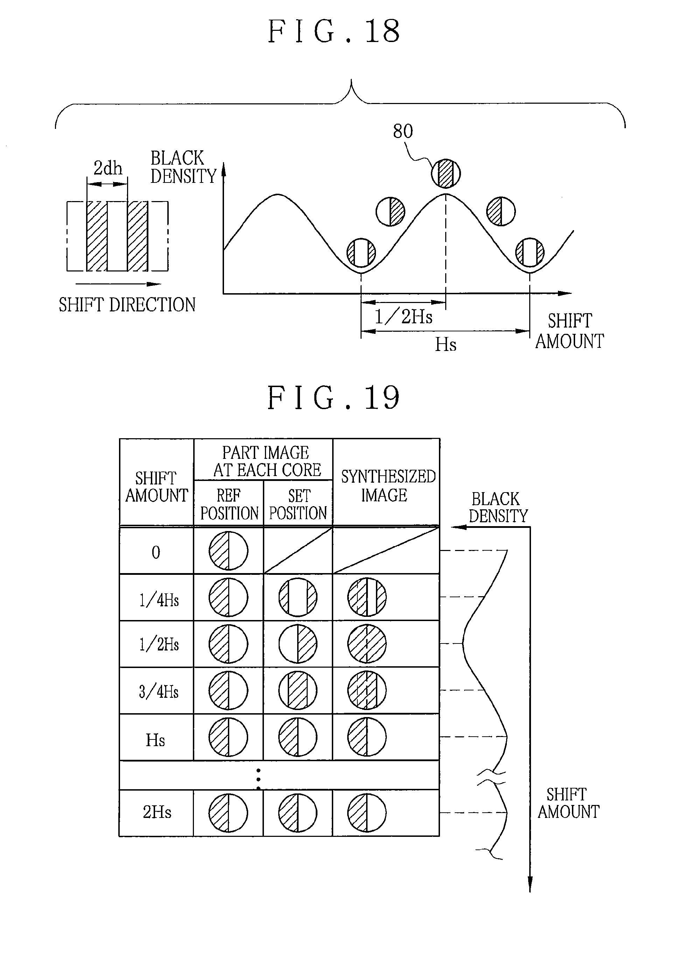

As the hood device 90 is positioned relative to the head assembly 15, a shift direction of the fiber optic image guide 31 is disposed with a width direction of the black and white areas 110a and 110b on the calibration chart 101. In Fig. 18, the black density changes according to the shift amount. The black density becomes the maximum when one of the black areas 110a is located at the center of the part image 80 transmitted by only one of the cores 50, and becomes the minimum when one of the white areas 110b is located at the center of the part image 80. If the black and white areas 110a and 110b are captured to appear in the part image 80 equally to one another, the black density becomes a medium value. As a frequency of the change of the black density is 2dh = kh.Hs, the shift amount is equal to the shift amount Hs. Although the part images 80 obtained with the cores 50 do not necessarily change equally to one another by way of test images, results of changes in the black density are approximately the same with minor local fluctuations.

When the piezoelectric actuator material 35 is driven appropriately, the part images 80 of the set positions and synthesized images of the part images 80 of the reference position and the set positions are in the forms of Fig. 19. In the example of the drawing, the black and white areas 110a and 110b appear equally in the part image 80 in the reference position. When the piezoelectric actuator material 35 is driven with the driving voltage 1/4.V and the distal tip 48 of the fiber optic image guide 31 is shifted by the shift amount 1/4.Hs, one of the white areas 110b is located at the center of the part image 80. One of the black areas 110a extends largely in the synthesized image except for a local part on the right side near to the center. In the case of driving with the driving voltage 1/2.V and shift amount 1/2.Hs, the black and white areas 110a and 110b appear equally and symmetrically in the part image 80 in the reference position. One of the black areas 110a extends fully in the synthesized image. In the case of driving with the shift amount 3/4.Hs, one of the black areas 110a is located at the center of the part image 80 in contrast with the result according to 1/4.Hs. One of the black areas 110a extends largely in the synthesized image except for a local part on the right side near to the right end. In the case of driving with the shift amount Hs, the same black or white pattern as the part image 80 of the reference position is obtained. The same black or white pattern in the synthesized image is obtained. If the shift amount becomes two times, three times and so on as much as Hs, the same black or white pattern as the part image 80 of the reference position is obtained. The same black or white pattern in the synthesized image is obtained.

The black density of the synthesized image is the maximum when the shift amount is 1/2.Hs for a full black area, and is the minimum when the shift amount is Hs (2Hs, 3Hs and so on) in the reference position. The black density of the synthesized image fluctuates periodically on a curve according to the shift amount at a period of Hs. Even though black and white areas in the part image 80 of the reference position do not appear equally to one another, the black density of the synthesized image in the reference position becomes equal to that of the synthesized image with the shift amount Hs only with a phase difference.

The CPU 62 detects black density in each of the test image according to the reference position and a synthesized image (not the synthesized image Gc) of the test image of the respective set positions. When the piezoelectric actuator material 35 is driven by the default driving voltage V, the distal tip 48 of the fiber optic image guide 31 is found to have been shifted at the shift amount Hs if the black density of the synthesized image is found equal to that of the test image according to the reference position. It is unnecessary to adjust the driving voltage of the piezoelectric actuator material 35.

If the black density of the synthesized image is unequal to that of the test image of the reference position, the distal tip 48 of the fiber optic image guide 31 does not displace with the constant shift amount Hs even upon driving the piezoelectric actuator material 35 with the default driving voltage V. The CPU 62 determines the driving voltage of the piezoelectric actuator material 35 to calibrate the shift amount. Specifically, the driving voltage is changed stepwise by 1/10.V near to the default driving voltage V, while the distal tip 48 of the fiber optic image guide 31 is loved back and forth from the reference position (for example, is moved with 8/10.V from the reference position, returned to the reference position, and moved with 9/10.V, and returned to the reference position in a repeated manner). The calibration chart 101 is captured by the CCD group 58 to determine a driving condition of the piezoelectric actuator material 35 with the driving voltage upon coincidence of the black density of the synthesized image with that of the test image of the reference position.

When the piezoelectric actuator material 35 is driven at the default driving voltage V, black density of the synthesized image may not be equal to that of the test image of the reference position. Then the CPU 62 writes information of a first driving voltage to a ROM 47 in the endoscope 5 such as an EEPROM or other rewritable storage (See Fig. 7), the first driving voltage being obtained by finely changing the voltage above and below the voltage V and by retrieving a level upon coincidence in the black density. At first, the ROM 47 stores the default driving voltage V as a driving condition. If the driving voltage is changed to a level different from V by the calibration, the CPU 62 rewrites the voltage in the ROM 47. The voltage is read by the CPU 62 of the processing apparatus 6 from the ROM 47 for use, and applied to the piezoelectric driver 61.

In conclusion, the shift amount of the fiber optic image guide 31 can be calibrated in the course of endoscopic examination as the calibration chart 101 is attached to the rear surface 91a of the cover door 91 for the calibrating operation. Occurrence of artifacts of the synthesized image Gc can be prevented.

In one preferred example of the endoscope, a light control mirror or dimming mirror is disposed on a rear surface of the cover door in place of or in addition to the calibration chart. The light control mirror, when the cover door is closed, is transparent, and when the cover door is open, is set reflective. A component of image light, which would not be transmitted only in the field of the imaging window 25, is conducted to the imaging window 25 by the light control mirror. Thus, it is possible to maintain the field of view when the cover door is closed, and to widen the field when the cover door is open.

It is possible to finish the resilient cap portion 20 or the cover door 91 in the hydrophilic surface finish for the anti-fogging purpose in a condition of high humidity in a human body. Examples of materials for the hydrophilic surface finish are a synthetic resin coating containing fine particles of hydrophilic polymer, and photocatalytic coating of titanium oxide, and the like.

The hood device or protection device or cover of the invention can be used repeatedly by washing periodically, or can be used only one time and discarded. In a case of a single use type of hood device, a removing mechanism is associated with the control wire. The hood device is exchanged at each time of the use.

Although the winder 17 is used in the above embodiment, other moving mechanisms can be used. For example, a pull ring may be formed on an end of the control wire for an operator manually to pull the same. Also, a moving mechanism may include a wheel for unwinding and pulling the control wire in a manner similar to a well-known steering wheel disposed in an endoscope for bending up or down a steering portion of the elongated tube. Furthermore, a MEMS motor as a microactuator can be used in place of the control wire to move the hood device from the closed position to the open position.

In the above embodiment, the resilient cap portion 20 and the cover door 91 for covering the distal end face 15a are formed from transparent material. However, the resilient cap portion 20 or the cover door 91 can be formed from colored material or opaque material. Although an object cannot be imaged in the course of advance of the endoscope, it is possible to guide the advance of the endoscope by monitoring a location of the endoscope by use of other imaging system, such as an X ray imaging system or the like.

A displacing device may be shaped in a form other than the rod form, for example, in a form of a quadrilateral prism. A fiber optic image guide is inserted in and attached to a support casing by an adhesive agent (not shown) or the like. A piezoelectric actuator material is overlaid on four faces of the support casing by way of electrodes. The fiber optic image guide is shifted together with the support casing up and down and to the right and left. For example, the displacing device displaces from the initial position in the leftward direction by 90 degrees at an amount of (3^{1/2}) /4. P to come to the set position with a first shift event. After this, the displacing device is returned to the initial position, and displaces in the downward direction at an amount of 1/4.P to come to the set position with a second shift event. The displacing device is returned from the set position of the second shift event to the initial position, and displaces to the right and upwards, and then is returned to the initial position again. Thus, the core 50 displaces in the two-dimensional path of a crossed shape by fine displacement. For the calibration, two calibration charts can be prepared for vertical and horizontal directions. A width of each of black and white areas can be set a constant value times the shift amount in the vertical and horizontal directions from an initial position.

However, there is a characteristic of the hysteresis in the piezoelectric actuator materials, of which the set position may be offset upon driving the piezoelectric actuator materials without a patterned manner. Thus, the displacing device is caused to displace with the same two-dimensional path and in the same sequence. In short, a sequence of driving the piezoelectric actuator materials for actuating the displacing device is set equal for every event. Also, a sequence of supplying electrodes with voltage on a couple of the right and left sides is kept the same. To calibrate the shift amount, the displacement is carried out with the same path in the same sequence.

As the fiber optic image guide is displaced by tilt of a root portion of the shift mechanism, the fiber optic image guide is likely to vibrate and stop with lag in the set positions without immediate stop. Thus, it is preferable with a piezoelectric driver to drive the piezoelectric actuator material or to use other anti-vibration methods in order to tilt the shift mechanism instantaneously in reverse to the displacing after a stop of the displacing device. Specifically, reaction force is previously measured by simulation or experimentally. An offset voltage for the piezoelectric actuator material is stored in a ROM. The piezoelectric control unit reads the information of the offset voltage from the ROM and sets the information in the piezoelectric driver. Furthermore, non-conductive fluid with high viscosity can be charged in a lumen for an anti-vibration structure by utilizing damping effect.

In the above embodiment, shift time for the shift mechanism to displace to a succeeding set position is shorter than clearing time from previous completion of storing charge of the CCD until a succeeding start of storing charge. However, the shift time may be longer than the clearing time for the reason of various factors, which include a length, material or shift amount of the shift mechanism, performance of the piezoelectric actuator material, or the like. In considering that the weight of the fiber optic image guide is relatively large, the shift time is very likely to be longer than the clearing time.

While the shift mechanism is set in the set position, the CCD driver is controlled by the CPU of the processing apparatus and supplies the CCD with an electronic shutter pulse. A start of storing charge is delayed. When the shift mechanism stops in the set position, storing charge is started. Otherwise, the light source is turned off while the shift mechanism is set in the set position, and then turned on when the shift mechanism stops in the set position.

In order to drive the CCD with reference to time required for shift to a succeeding one of the set positions, the frame rate must be set small should the shift time be longer than the clearing time. However, it is possible without blur to obtain an image even with the presently high frame rate by sweeping out charge with an electronic shutter pulse, or by turning off the light source, or by other methods.

In the above embodiment, the image synthesizing unit synthesizes the image only when the composite imaging mode is designated. However, it is possible to synthesize an image also in the normal imaging mode. This is effective in compensating for a loss region of the cladding even though an image for reflecting an object image positioned in association with the cladding cannot be obtained. Image quality can be high.

In the above embodiment, the image synthesizing unit carries out synthesis for one two-dimensional shift sequence, to output one synthesized image. However, a problem occurs in that a frame rate of the composite imaging mode is lower than the normal imaging mode. To solve this problem, it is preferable to raise a frame rate to four times as high a level as the frame rate for the normal imaging mode, namely change over the frame rate in response to designating the composite imaging mode.

Specifically, frequency of the clock signal of the system clock in the CPU 62 is changed to change frequency of a drive signal of the CCD driver 60. Otherwise, it is possible in the CCD driver 60 to dispose a clock divider without changing the clock signal. The clock signal can be divided by the clock divider to change the frequency.

In another variant of the embodiment of the four shift events, the images GO-G3 of one two-dimensional shift sequence is used to create a synthesized image Gc. Then the images GO-G3 and one image G0 according to a second two-dimensional shift sequence can be used to create a synthesized image Gc. Similarly, a combination of images Go-G3 for one two-dimensional shift sequence can be changed by one image. An earliest one of the images GO-G3 is replaced with a newest one of images GO-G3 so as to create a series of synthesized images Gc. This is effective in preventing complexity in the control by changing a period of a clock signal. A drop of the frame rate can be prevented.

Elements of the hardware can be incorporated in a housing separate from the processing apparatus, or may be incorporated in the endoscope, the hardware including the three-CCD assembly, the input interface for setting the imaging mode and the number of shift events, and electronic circuits to constitute the image synthesizing unit, sync control unit and piezoelectric control unit.

One preferred light source apparatus can include a blue laser light source, which may have a characteristic with a central wavelength of 445 nm. A wavelength conversion device is disposed on an exit side of the light guide devices 27 at its distal end. The wavelength conversion device contains plural phosphors which partially absorb the laser light from the blue laser light source, to emit converted light with colors from green to yellow by excitation. The laser light from the blue laser light source and the excitation light from green to yellow after the conversion are coupled together, to produce white light of high brightness. It is possible to obtain sufficiently bright light with a small number of light guide devices, namely one or two, because white light can be delivered with higher brightness than the former embodiment. The diameter of the endoscope can be reduced more effectively for an ultra thin tube.

Note that a single CCD assembly may be used instead of a three-CCD assembly. In the above embodiments, the first connector 10 is used commonly for the connection of the fiber optic image guide and the connection cable to the processing apparatus. However, two separate connectors may be used and may contain the fiber optic image guide and the connection cable discretely.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. An endoscope for image pickup of an object by receiving image light with an image sensor (56), comprising:
an objective lens system (30), incorporated in an elongated tube (8), for entry of said image light from said object;
a fiber optic image guide (31), including a plurality of optical fibers being bundled, and having a distal tip (48), being inserted through said elongated tube, for transmitting said image light focused on said distal tip by said objective lens system in a proximal direction;
a piezoelectric actuator (35), disposed on an outer side of said distal tip, for periodically displacing said distal tip, wherein said image sensor picks up said image light from said fiber optic image guide for plural times in synchronism with displacement, to form one synthesized image;
a hood device (16, 90), mounted on a head assembly (15) of said elongated tube, and shiftable between a closed position for covering a distal end face of said head assembly and an open position for revealing said distal end face;
a moving mechanism (17, 23, 94) for moving said hood device from said closed position to said open position.

2. An endoscope as defined in claim 1, wherein said hood device includes a transparent cover portion for covering said distal end face.

3. An endoscope as defined in claim 2, wherein said cover portion is a resilient cap portion including an end opening;
said hood device includes a sleeve, disposed about said distal tip to extend from said resilient cap portion in said proximal direction, moved between said open and closed positions by said moving mechanism, for narrowing said end opening in front of said distal end face when in said closed position, and for widening said end opening by pushing open said resilient cap portion with said distal end face when in said open position.

4. An endoscope as defined in claim 2, wherein said cover portion is a cover door, and is moved by said moving mechanism for opening and closing.

5. An endoscope as defined in claim 1, further comprising a calibration chart, secured to a surface of said cover door directed in said proximal direction, for calibrating a shift amount of said fiber optic image guide.

6. An endoscope as defined in claim 5, wherein said calibration chart includes plural stripe areas extending in a predetermined direction;
said distal tip is displaced back and forth in a shift direction extending across said predetermined direction, and picks up an image of said calibration chart to form a test image, said shift amount being calibrated according thereto.

7. An endoscope as defined in claim 6, wherein said distal tip is displaced by application of a driving voltage to said piezoelectric actuator;
said test image is evaluated for calibration so as to determine an adjusted driving voltage for displacement of imaging.

8. An endoscope as defined in claim 1, wherein said moving mechanism includes:
a control wire secured to said hood device;
a wire moving device, disposed on a side of said proximal direction, for winding or unwinding said control wire.

9. An endoscope as defined in claim 8, wherein said control wire is embedded partially in said elongated tube.
